(19) Europäisches Patentamt
European Patent Office
Office européen des brevets

(11) Veröffentlichungsnummer: **0 027 776**
**B1**

(12) **EUROPÄISCHE PATENTSCHRIFT**

(45) Veröffentlichungstag der Patentschrift: **02.05.85**

(21) Anmeldenummer: **80810287.5**

(22) Anmeldetag: **12.09.80**

(51) Int. Cl.⁴: **C 07 F 9/165,** C 07 F 9/173, C 07 F 9/21, C 08 K 5/51, C 10 M 137/00

(54) **Phenole als Stabilisatoren und sie enthaltende Zusammensetzungen.**

(30) Priorität: **18.09.79 CH 8417/79**

(43) Veröffentlichungstag der Anmeldung: **29.04.81 Patentblatt 81/17**

(45) Bekanntmachung des Hinweises auf die Patenterteilung: **02.05.85 Patentblatt 85/18**

(84) Benannte Vertragsstaaten: **BE DE FR GB IT NL**

(56) Entgegenhaltungen:
**FR - A - 1 482 059**
**FR - A - 2 091 662**
**FR - A - 2 136 589**
**GB - A - 1 232 039**
**US - A - 3 708 520**

**CHEMICAL ABSTRACTS, Band 91, Nr. 14, 1. Oktober 1979, Seite 45, Nr. 108838x Columbus, Ohio, U.S.A. A.M.A. AMARAPATHY et al.: "Rubberbound antioxidants by reactions of natural in the latex"**
**CHEMICAL ABSRTRACTS, Band 91, formula index, C16-Z, 1979, * Formel C19H28O3, Benzenepropanoic acid ***
**CHEMICAL ABSTRACTS, Band 91, Nr. 16, 15. Oktober 1979, Seite 29, Nr. 124371v Columbus, Ohio, U.S.A. E.S.**

(73) Patentinhaber: **CIBA-GEIGY AG, Postfach, CH-4002 Basel (CH)**

(72) Erfinder: **Rosenberger, Siegfried, Im Gehracker 11, CH-4125 Riehen (CH)**

(56) Entgegenhaltungen: (Fortsetzung)
**TORSUEVA et al.: "Comparative stability of differebt polyeolfins under inhibited oxidation conditions"**

***Die Akte enthält technische Angaben, die nach dem Eingang der Anmeldung eingereicht wurden und die nicht in dieser Patentschrift enthalten sind.***

**EP 0 027 776 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft neue Phenole, deren Verwendung als Stabilisatoren für organisches Material, insbesondere Schmiermittel, deren Herstellung, sowie das mit den neuen Verbindungen stabilisierte organische Material.

Phenolgruppen enthaltende Antioxidans-Stabilisatoren für organisches Material, insbesondere Polymere, sind bekannt. Ferner ist aus US Pat. Nr. 3 745 148 und US Pat. Nr. 3 017 586 die Verwendung von Phenol- und Dithiophosphatgruppen enthaltenden Stabilisatoren für Schmiermittel bekannt. Weiter ist bereits durch C.A. 91 (1979) Nr. 108 838 x und US-Patent 3 708 520 bekannt, 3,5-Dialkyl-4-hydroxy-phenyl-propionsäurevinylester als Antioxidantien einzusetzen. Auch die Verwendung von Dithiophosphatestern von Carbonsäuren als Antioxidantien in Schmiermitteln ist bekannt aus dem FR-Patent 1 482 059. Die beschriebenen Verbindungen genügen aber nicht den hohen Anforderungen, welche an ein Hochdruck- und Antiwear-Additiv gestellt werden.

Es wurde nun eine neue Klasse phenolhaltiger Verbindungen gefunden, die in organischen Materialien eine hohe Farb- und Extraktionsstabilität bewirken und in Schmiermitteln neben guter Antioxidations- und Antikorrosionswirkung ausgezeichnete Extreme pressure- und Antiweareigenschaften entfalten. Daneben zeichnen sich die neuen Verbindungen in Schmiermitteln durch geringe Schlammbildung und keine Aschebildung aus.

Die Verbindungen entsprechen der allgemeinen Formel I

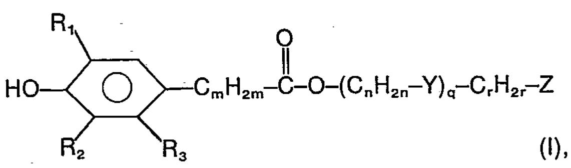

(I),

worin $R_1$ und $R_2$ unabhängig voneinander $C_1$–$C_{12}$ Alkyl, gegebenenfalls mit 1 bis 3 Alkylgruppen mit insgesamt 1 bis 12 C-Atomen substituiertes Phenyl, $C_7$–$C_9$ Aralkyl oder $C_5$–$C_7$ Cycloalkyl bedeuten, und $R_2$ zusätzlich Wasserstoff oder Chlor ist, und $R_3$ Wasserstoff oder Methyl bedeutet, und m 0, 1, 2, 3 oder 4 ist, und n und r unabhängig voneinander eine ganze Zahl von 0 bis 20 und q 1, 2 oder 3 bedeutet, mit der Bedingung, dass $(n{\cdot}q)+r$ eine ganze Zahl von 0 bis 24 ist, und Y eine Gruppe der Formel II oder III

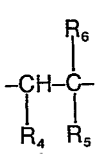

(II),

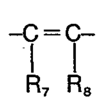

(III)

bedeutet, worin $R_4$ und $R_5$ Wasserstoff oder Hydroxy sind, und mindestens einer der Reste $R_4$ und $R_5$ eine Gruppe der Formel IV

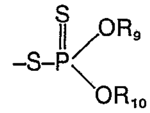

(IV)

darstellt, und $R_6$ Wasserstoff oder Methyl ist, und einer der Reste $R_7$ und $R_8$ Wasserstoff und der andere eine Gruppe der Formel IV bedeutet, wobei $R_9$ und $R_{10}$ unabhängig voneinander $C_1$–$C_{30}$ Alkyl, $C_2$–$C_{10}$ Alkoxyalkyl oder gegebenenfalls mit 1 bis 3 Alkylgruppen mit insgesamt 1 bis 12 C-Atomen substituiertes Phenyl, $C_7$–$C_9$ Aralkyl oder $C_5$–$C_7$-Cycloalkyl bedeuten, oder $R_9$ und $R_{10}$ zusammen eine Gruppe der Formel V

$$-(R_{11})C(R_{12})-[(R_{13})C(R_{14})]_t-(R_{15})C(R_{16})- \quad (V)$$

bedeuten, worin t 0 oder 1 ist und $R_{11}$, $R_{12}$, $R_{13}$, $R_{14}$, $R_{15}$ und $R_{16}$ unabhängig voneinander Wasserstoff oder Methyl sind, und Z Wasserstoff oder eine Gruppe der Formel VI

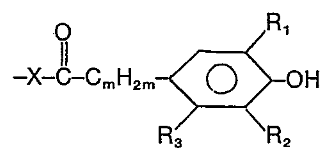

(VI),

worin die Symbole $R_1$, $R_2$, $R_3$ und m die oben angegebene Bedeutung haben, ist.

$R_1$ und $R_2$ sind als $C_1$–$C_{12}$ Alkyl z.B. Methyl, Äthyl, iso-Propyl, sec.Butyl, t.Butyl, t.Amyl, n-Hexyl, 1,1,3,3-Tetramethylbutyl oder 1,1,3,3,5,5-Hexamethylhexyl. Bevorzugte Alkylgruppen besitzen 1 bis 8 C-Atome. $R_1$ ist ferner in bevorzugten Verbindungen t.Butyl, t.Amyl oder 1,1,3,3-Tetramethylbutyl.

Sind $R_1$, $R_2$, $R_9$ und $R_{10}$ $C_7$–$C_9$ Aralkyl, so kann es sich um Benzyl, α-Phenyläthyl oder α,α-Dimethylbenzyl handeln.

Bedeuten $R_1$, $R_2$, $R_9$ und $R_{10}$ $C_5$–$C_7$ Cycloalkyl, so handelt es sich um Cyclopentyl, Cyclohexyl oder Cycloheptyl.

$R_1$, $R_2$, $R_9$ und $R_{10}$ können als Phenyl, Aralkyl und Cycloalkyl mit 1 bis 3 Alkylgruppen mit insgesamt 1 bis 12 C-Atomen substituiert sein. Beispiele solcher Alkylsubstituenten sind Methyl, Äthyl, Isopropyl, n-Butyl, sec.Butyl, t.Butyl, n-Hexyl, 1,1,3,3-Tetramethylbutyl, n-Nonyl, 1,1,3,3,5,5-Hexamethylhexyl oder n-Dodecyl.

Die bevorzugte Bedeutung von $R_3$ ist Wasserstoff und von m 1 oder 2.

Bei der Gruppe $-C_m H_{2m}$ kann es sich um eine verzweigte, bevorzugt jedoch geradkettige Alkylengruppe handeln.

n und r können unabhängig voneinander eine ganze Zahl von 0 bis 20 und bevorzugt 0 bis 8 bedeutet. In bevorzugten Verbindungen bedeutet n 0 bis 6 und insbesondere 1 und r ist bevorzugt 1 und insbesondere 0.

Die Gruppen $-C_nH_{2n}$- und $-C_rH_{2r}$- können geradkettige oder verzweigte Alkylgruppen sein. Beispiele solcher Gruppen sind: Methylen, Äthylen-1,2; Propylen-1,3; Butylen-1,4; Pentamethylen-1,5; Hexamethylen-1,6; Octamethylen-1,8; Nonamethylen-1,9; 1-Propyliden, Isopropyliden, 1-Methyläthylen-1,2; 1-Äthyliden, 1-Butyliden, 2-Butyliden, 2-Pentyliden, 1-Pentyliden, 1-Hexyliden, 3-Methylpentamethylen-1,5; 4,8,12-Trimethyl-tridecamethylen-1,13.

q kann 1, 2 oder 3 sein. Die bevorzugte Bedeutung ist 1. Bedeutet q 2 bzw. 3, so müssen die 2 bzw. 3 Einheiten der Formel $-(C_nH_{2n}-Y)-$ nicht identisch sein. Beispiele von Brückengliedern der Formel $-(C_nH_{2n}-Y)_q-$ sind $-Y-CH_2-Y-$, $-CH_2-Y-Y-$, $-(CH_2)_8-Y-CH_2-Y-$, $-(CH_2)_8-Y-CH_2-Y-CH_2-Y-$.

Die erfindungsgemässen Verbindungen erfüllen die Bedingung, dass die Summe von $(n \cdot q)+r$ eine Zahl von 0 bis 24, bevorzugt 0 bis 16 und insbesondere 0 bis 6 bedeutet.

Eine bevorzugte Bedeutung von Y ist die Gruppe der Formel II. Darin muss mindestens einer der Reste $R_4$ und $R_5$ eine Gruppe der Formel IV, der andere Hydroxy oder bevorzugt Wasserstoff sein. Es gehören jedoch auch Verbindungen, in welchen sowohl $R_4$ wie $R_5$ eine Gruppe der Formel IV sind, wobei die in beiden Gruppen vorkommenden Substituenten $R_9$ und $R_{10}$ bevorzugt die gleiche Bedeutung haben, zur vorliegenden Erfindung.

Die bevorzugte Bedeutung von $R_6$ ist Wasserstoff.

Sind $R_9$ und $R_{10}$ $C_1-C_{30}$ Alkyl, so kann es sich z.B. um Methyl, Äthyl, n-Propyl, Isopropyl, n-Butyl, sec.Butyl, t.Butyl, n-Hexyl, n-Octyl, 2-Äthylhexyl, 6-Methylheptyl, n-Octyl oder geradkettiges oder verzweigtes Nonyl, Decyl, Dodecyl, Tridexyl, Tetradexyl, Octadecyl, Eicosyl, Docosyl, Tetracosyl oder Triacontyl handeln. Besonders bei langkettigen Alkylgruppen handelt es sich in der Regel um Isomerengemische.

Bedeuten $R_9$ und $R_{10}$ $C_2-C_{10}$ Alkoxyalkyl, so kann der Alkylteil 1 bis 3 C-Atome enthalten und der Alkoxyteil aus 1–8 C-Atomen bestehen, wie z.B. in Methoxymethyl, Äthoxymethyl, 2-Methoxyäthyl, 2-Äthoxyäthyl, 2-n-Butoxyäthyl, 3-n-Butoxypropyl, 2-Octoxyäthyl oder Methoxypropyl.

Bedeuten $R_9$ und $R_{10}$ zusammen eine Alkylengruppe der Formel V, so handelt es sich beispielsweise um Äthylen-1,2; 1-Methyläthylen-1,2; 1,1-Dimethyläthylen-1,2; 1,1,2-Trimethyläthylen-1,2; 1,1,2,2-Tetramethyläthylen-1,2; Propylen-1,3; 1-Methylpropylen-1,3; 1,1,3-Trimethylpropylen-1,3 oder 2,2-Dimethylpropylen-1,3.

Die bevorzugte Bedeutung von Z ist Wasserstoff.

Bevorzugte Verbindungen entsprechen der Formel I, worin $R_1$ und $R_2$ unabhängig voneinander $C_1-C_8$ Alkyl sind und $R_2$ zusätzlich Wasserstoff bedeutet und $R_3$ Wasserstoff oder Methyl ist, und m 1 oder 2 ist, und n und r unabhängig voneinander eine ganze Zahl von 0 bis 8 und q 1 ist, mit der Bedingung, dass $(n \cdot q)+r$ eine ganze Zahl von 0 bis 16 bedeutet, und Y eine Gruppe der Formel II oder III ist, worin $R_4$ und $R_5$ Wasserstoff oder Hydroxy sind, und mindestens einer der Reste $R_4$ und $R_5$ eine Gruppe der Formel IV darstellt, und $R_6$ Wasserstoff ist, einer der Reste $R_7$ und $R_8$ Wasserstoff, und der andere eine Gruppe der Formel IV bedeutet, wobei $R_9$ und $R_{10}$ unabhängig voneinander $C_1-C_{22}$-Alkyl sind, oder $R_9$ und $R_{10}$ zusammen eine Gruppe der Formel V bedeuten, worin die Symbole t und $R_{11}$ bis $R_{16}$ die oben angegebene Bedeutung haben, und Z Wasserstoff ist.

Besonderes Interesse gilt auch Verbindungen der Formel I, worin $R_1$ t.-Butyl, t.-Amyl oder 1,1,3,3-Tetramethylbutyl ist und $R_2$ $C_1-C_8$ Alkyl bedeutet, $R_3$ Wasserstoff ist, und m 1 oder 2, und X –O– ist, und n eine ganze Zahl von 0 bis 6 bedeutet, und r 0 oder 1, und q 1 ist, und Y eine Gruppe der Formel II oder III ist, worin einer der Substituenten $R_4$ und $R_5$ Wasserstoff und der andere eine Gruppe der Formel IV ist, wobei $R_9$ und $R_{10}$ die oben angegebene Bedeutung haben.

In der untenstehenden Tabelle sind Beispiele von Verbindungen der Formel I zusammengestellt:

Typische Beispiele der erfindungsgemässen Verbindungen entsprechend den Formeln VII oder VIII:

$$A-C_mH_{2m}-\overset{O}{\overset{\|}{C}}-X-C_nH_{2n}-\underset{}{CH}(R_4)-C(R_5)(R_6)-C_rH_{2r}-Z \qquad (VII)$$

| Nr. | A | m | $-C_mH_{2m}-$ | X | n | $-C_nH_{2n}$ | eines von $R_4$ und $R_5$ | und das andere | $R_6$ | r | $-C_rH_{2r}-$ | Z |
|---|---|---|---|---|---|---|---|---|---|---|---|---|
| 1. | 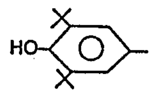 | 0 | | –S– | 0 | | H | $-S-P(S)(O-I-C_{13}H_{27})_2$ | H | 0 | | H |
| 2. | do. | 3 | $-CH_2-C(CH_3)_2-$ | –O– | 1 | $-CH_2-$ | H | 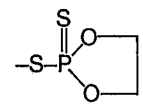 | H | 0 | | H |
| 3. | do. | 2 | $-CH(CH_3)-$ | –NH– | 2 | $-CH_2CH_2-$ | –OH | $-S-P(S)(O-I-C_3H_7)_2$ | H | 0 | | H |
| 4. | do. | 2 | $-CH_2CH_2-$ | –O– | 3 | $-(CH_2)_3-$ | H | $-S-P(S)(O-C_{22}H_{45})_2$ | H | 0 | | H |
| 5. | do. | 1 | $-CH_2-$ | –O– | 3 | $-CH(CH_3)CH_2-$ | H | $-S-P(S)(O-I-C_8H_{17})_2$ | H | 0 | | H |
| 6. | do. | 2 | $-CH_2CH_2-$ | –O– | 2 | $-CH(CH_3)-$ | H | $-S-P(S)(O-I-C_4H_9)_2$ | H | 1 | $-CH_2-$ | H |
| 7. | do. | 1 | $-CH_2-$ | –O– | 4 | $-CH(CH_2CH_2CH_3)-$ | H | 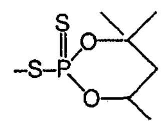 | H | 1 | $-CH_2-$ | H |
| 8. | do. | 4 | $-(CH_2)_4$ | –O– | 4 | $-(CH_2)_4$ | –OH | $-S-P(S)(OC_{12}H_{25})_2$ | H | 0 | | H |
| 9. | do. | 2 | $-CH_2CH_2-$ | –O– | 6 | $-CH_2CH_2CH(CH_3)-CH_2-CH_2-$ | H | 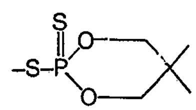 | $CH_3$ | 1 | $-CH_2-$ | H |
| 10. | do. | 2 | $-CH_2CH_2-$ | –O– | 8 | $-(CH_2)_8-$ | H | $-S-P(S)(O-I-C_{13}H_{27})_2$ | H | 8 | $-(CH_2)_8-$ | H |
| 11. | do. | 2 | $-CH_2CH_2-$ | –O– | 1 | $-CH_2-$ | 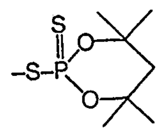 | 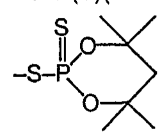 | H | 1 | $-CH_2-$ | H |
| 12. | do. | 1 | $-CH_2-$ | –O– | 1 | $-CH_2-$ | $-S-P(S)(O-I-C_3H_7)_2$ | $-S-P(S)(O-I-C_3H_7)_2$ | H | 0 | | H |
| 13. | do. | 2 | $-CH_2CH_2-$ | –O– | 1 | $-CH_2-$ | $-S-P(S)(O-I-C_3H_7)_2$ | $-S-P(S)(O-I-C_3H_7)_2$ | H | 1 | $-CH_2-$ | 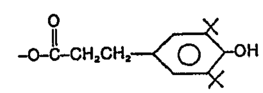 |
| 14. | 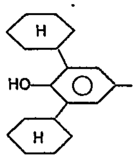 | 2 | $-CH_2CH_2-$ | –O– | 1 | $-CH_2-$ | H | 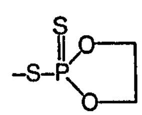 | H | 0 | | H |

| | | | | | | | | | | | | |
|---|---|---|---|---|---|---|---|---|---|---|---|---|
| 15. | 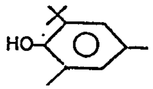 | 1 | $-CH_2-$ | $-NH-$ | 1 | $-CH_2-$ | $-OH$ | $-S-P(S)(0-C_9H_{19})_2$ | H | 0 | | H |
| 16. | do. | 2 | $-CH_2CH_2-$ | $-O-$ | 1 | $-CH_2-$ | $-S-P(S)(O-I-C_8H_{17})_2$ | $-S-P(S)(O-I-C_8H_{17})_2$ | H | 1 | $-CH_2-$ | H |
| 17. | 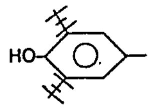 | 2 | $-CH_2CH_2-$ | $-O-$ | 1 | $-CH_2-$ | H | $-S-P(S)(O-I-C_4H_9)_2$ | H | 0 | | H |
| 18. | 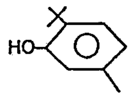 | 2 | $-CH_2CH_2-$ | $-O-$ | 1 | $-CH_2-$ | H | $-S-P(S)(O-I-C_{13}H_{27})_2$ | H | 0 | | H |

$$A-C_mH_{2m}-\overset{O}{\overset{\|}{C}}-X-C_2H_{2n}-\overset{R_7}{\overset{|}{C}}=\overset{R_8}{\overset{|}{C}}-C_rH_{2r}-Z \qquad (VIII)$$

| Nr. | A | m | $-C_mH_{2m}-$ | X | n | $-C_nH_{2n}$ | eines von $R_7$ und $R_8$ | und das andere | r | $-C_rH_{2r}-$ | Z |
|---|---|---|---|---|---|---|---|---|---|---|---|
| 19. | 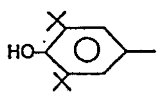 | 1 | $-CH_2-$ | $-O-$ | 1 | $-CH_2-$ | H | $-S-P(S)(O-I-C_3H_7)_2$ | 0 | | H |
| 20. | do. | 2 | $-CH_2CH_2-$ | $-O-$ | 1 | $-CH_2-$ | H | $-S-P(S)(O-I-C_8H_{17})_2$ | 1 | $-CH_2-$ | H |
| 21. | do. | 2 | $-CH_2CH_2-$ | $-O-$ | 0 | | H | 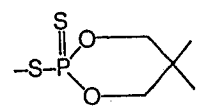 | 0 | | H |
| 22. | 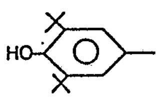 | 2 | $-CH_2CH_2-$ | $-O-$ | 1 | $-CH_2-$ | H | H | 0 | | H |
| 23. | do. | 2 | $-CH_2CH_2-$ | $-O-$ | 1 | $-CH_2-$ | eine weitere C–C-Bindung mit $R_7$ bzw. $R_8$ | eine weitere C–C-Bindung mit $R_7$ bzw. $R_8$ | 0 | | H |

Die Herstellung der Verbindungen der Formel I erfolgt auf an sich bekannte Weise, z.B. durch Veresterung einer Carbonsäure oder eines reaktionsfähigen Derivats, wie Säurechlorid oder Niederalkylester der Formel IX

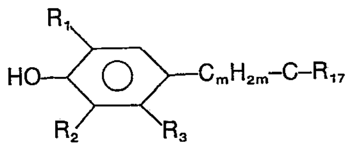

(IX),

worin $R_{17}$ OH, Cl oder $C_1$–$C_4$ Alkoxy bedeutet, und die übrigen Symbole die oben angegebene Bedeutung haben, mit einem Hydroxyalken, -epoxid oder -alkin der Formel X

$$HO-(C_nH_{2n}-Y^*)_q-C_rH_{2r}-Z \quad (X),$$

worin Y* eine Gruppe $-CH=C(R_6)-$, 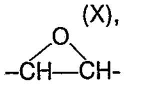 oder $-C\equiv C-$ bedeutet, und die übrigen Symbole der Formel X und $R_6$ die oben angegebene Bedeutung haben. Zur auf diese Weise erhaltenen Verbindung der Formel XI

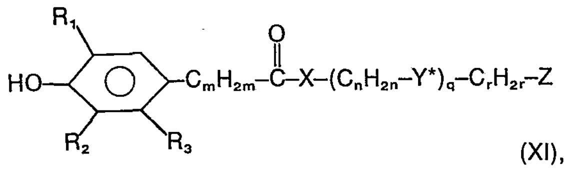

(XI),

worin die Symbole die oben angegebene Bedeutung haben, addiert man eine Verbindung der Formel XII

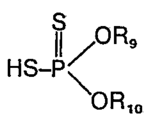

(XII),

worin $R_9$ und $R_{10}$ die oben angegebene Bedeutung haben.

Die Addition kann in einem inerten Lösungsmittel oder vorzugsweise ohne Lösungsmittel bei Temperaturen von 0–180 °C, vorzugsweise ohne Lösungsmittel bei Temperaturen von 0–180 °C, vorzugsweise 50–150 °C, und gegebenenfalls in Gegenwart eines Radikal-Initiators durchgeführt werden. Als Lösungsmittel eignen sich gegebenenfalls chlorierte aliphatische oder aromatische Kohlenwasserstoffe, wie Spezialbenzine, Hexan, Heptan, Methylenchlorid, 1,2-Dichloräthan, Benzol, Chlorbenzol, Dichlorbenzol, Toluol, Xylol; oder Äther, wie Diäthyläther, Dioxan oder Tetrahydrofuran.

Für die radikalisch katalysierte Additionsreaktion verwendbare Radikalinitiatoren sind z.B. insbesondere Per- und Azoverbindungen, oder UV-Licht. Gebräuchliche Perverbindungen sind Wasserstoffperoxid, Di-t.butylperoxid, Cumolhydroperoxid oder Dibenzoylperoxid. Als Azoverbindung eignet sich insbesondere α,α'-Azodiisobuttersäuredinitril.

Wird der Dithiophosphorsäure-O,O-diester der Formel XII an ein Alken der Formel XI, worin Y* $-CH=C(R_6)-$ bedeutet, und die übrigen Symbole die oben definierte Bedeutung haben, addiert, so entstehen Verbindungen der Formel I, worin Y eine Gruppe der Formel II ist. Es entsteht dabei neben derjenigen Verbindung, in der $R_4$ Wasserstoff ist und $R_5$ die Gruppe der Formel IV bedeutet, in der Regel die isomere Verbindung, in welcher $R_4$ eine Gruppe der Formel IV ist, und $R_5$ Wasserstoff bedeutet. Die Trennung der beiden Isomeren ist auf bekannte Weise möglich. Für die Anwendung als Schmiermittelzusätze wird jedoch in der Regel das Isomerengemisch eingesetzt.

Wird der Dithiophosphorsäure-O,O-diester der Formel XII an ein Epoxid der Formel XI, worin Y* 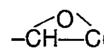 bedeutet, und die übrigen Symbole die oben angegebene Bedeutung haben, addiert, so entstehen Verbindungen, in welchen Y eine Gruppe der Formel II ist, worin eines von $R_4$ und $R_5$ Hydroxy ist, und das andere eine Gruppe der Formel IV bedeutet. Auch in diesem Fall entsteht in der Regel ein Isomerengemisch, welches, falls gewünscht, getrennt werden kann.

Wird der Dithiophosphorsäure-O,O-diester der Formel XII an ein Alkin der Formel XI, worin Y* $-C\equiv C-$ bedeutet, und die übrigen Symbole die oben angegebene Bedeutung haben, addiert, so entstehen bei der Umsetzung von ungefähr einem Mol der Verbindung der Formel XII mit ungefähr einem Mol der Verbindung der Formel XI, solche Vertreter der Formel I, in welchen Y eine Gruppe der Formel III ist, wobei einer der Reste $R_7$ und $R_8$ Wasserstoff ist, und der andere eine Gruppe der Formel IV bedeutet. Auch in diesem Falle entstehen wie oben Isomerengemische. Werden ungefähr zwei Mol einer Verbindung der Formel XII mit ungefähr einem Mol der Verbindung der Formel XI (Y*: $-C\equiv C-$) umgesetzt, so erhält man als Produkt Stoffe der Formel I, worin Y eine Gruppe der Formel II ist, worin $R_4$ und $R_5$ eine Gruppe der Formel IV bedeuten.

Die Dithiophosphate der Formel XII sind bekannte Verbindungen und vielfach handelsüblich.

Die erfindungsgemässen Verbindungen der Formel I sind wirksame Antioxydantien für organisches Material. Sie eignen sich für das Stabilisieren einer grossen Anzahl von organischen Polymeren. Beispiele für solche Polymeren sind:

1. Polymere, die sich von Mono- und Diolefinen ableiten, wie Polyäthylen, das gegebenenfalls vernetzt sein kann, Polypropylen, Polyisobutylen, Polymethylbuten-1, Polymethylpenten-1, Polyisopren, Polybutadien.

2. Mischungen der unter 1 genannten Homopolymeren, wie z.B. Gemische von Polypropylen und Polyäthylen, Polypropylen und Polybuten-1, Polypropylen und Polyisobutylen.

3. Copolymere der den unter 1 genannten Homopolymeren zugrundeliegenden Monomeren, wie Äthylen-Propylen-Copolymere, Äthylen-Buten-1-Copolymere, sowie Terpolymere von Äthylen und Propylen mit einem Dien, wie z.B. Hexadien, Dicyclopentadien oder Äthyliden.

4. Acrylnitril-Copolymere, wie z.B. Butadien-Acrylnitril-Methylmethacrylat-Copolymer und ABS.

5. Polystyrol und seine Copolymeren, wie SAN, IPS, ASA und EP modifizierte Styrolcopolymerisate.

6. Halogenhaltige Vinylpolymere.

7. Polyurethane.

8. Polycarbonate.

9. Polyamide.

10. Polyester.

Die Stabilisatoren können gegebenenfalls mit anderen Zusätzen kombiniert werden, wie andere Antioxydantien, Gleitmittel wie Castearat, Pigmente, Farbstoffe, UV-Absorber, sterisch gehinderte Amine als Lichtschutzmittel, Metalldesaktivatoren, Talk und andere Füllstoffe.

Im allgemeinen werden die erfindungsgemässen Stabilisatoren in 0,01 bis 5 Gew.-%, bezogen auf das stabilisierte Material, verwendet, wobei die Menge je nach Substrat und Anwendung verschieden sein kann. Bevorzugt sind 0,05 bis 2 Gew.-%, besonders bevorzugt sind 0,1 bis 1 Gew.-%.

Einige der erfindungsgemässen Stabilisatoren können auch auf die Polymere aufgepfropft werden (vgl. dazu DE-OS 2 509 654). Dabei werden die Stabilisatoren in Mengen von 0,01 bis 5 oder 10%, typischerweise 0,25 bis 3% und besonders 0,5 bis 2% bezogen auf das Gewicht des Polymers eingesetzt. Bei der Aufpfropfmethode wird zudem ein Radikalbildner zugefügt. Das Gewichtsverhältnis Stabilisator zu Radikalbildner beträgt 100:1 bis 0,25:1.

Die Verbindungen der Formel I wirken in Schmiermitteln schon in sehr geringen Mengen als Hochdruck-Zusätze. So zeigen mineralische und synthetische Schmieröle, sowie deren Gemische, welche mit 0,001 bis 5 Gew.-% bezogen auf das Schmiermittel, und vorzugsweise 0,02 bis 3 Gew.-% einer Verbindung der Formel I ausgestattet sind, ausgezeichnete Hochdruck-Schmiereigenschaften, welche durch stark reduzierte Abnutzungserscheinungen der zu schmierenden Reibpartner deutlich werden. Die in Frage kommenden Schmiermittel sind dem Fachmann geläufig und z.B. im «Schmiermittel Taschenbuch» (Hüthig-Verlag, Heidelberg, 1974) beschrieben.

Die Schmierölformulierung kann zusätzlich noch andere Additive enthalten, die zugegeben werden, um gewisse Grundöleigenschaften zu verbessern, wie Antioxidantien, Metallpassivatoren, Rostinhibitoren, Viskositätsindex-Verbesserer, Stockpunkterniedriger, Dispersants/Detergents und andere Verschleissschutz-Additive.

Beispiele für Antioxidantien sind:

(a) Alkylierte und nicht-alkylierte aromatische Amine und Mischungen davon, z.B.:

Dioctyldiphenylamin, Mono-t-octylphenyl-α- und -β-naphthylamine, Phenothiazin, Dioctylphenothiazin, Phenyl-α-naphthylamin, N,N'-Di-sec.butyl-p-phenylendiamin.

(b) Sterisch gehinderte Phenole, z.B. 2,6-Ditert.-butyl-p-cresol, 4,4'-Bis-(2,6-diisopropylphenol), 2,4,6-Triisopropylphenol, 2,2'-Thio-bis-(4-methyl-6-tert.-butyl-phenol), 4,4'-Methylen-bis-(2,6-di-tert.-butylphenol), Tetra-[methylen-3-(3,5-di-tert.-butyl-4-hydroxyphenyl)-propionat]-methan.

(c) Alkyl-, Aryl- oder Alkaryl-phosphite, z.B.:

Trinonylphosphit, Triphenylphosphit, Diphenyldecylphosphit.

(d) Ester von Thiodipropionsäure oder Thiodiessigsäure, z.B.:

Dilaurylthiodipropionat oder Dioctylthiodiacetat.

(e) Salze von Carbamin- und Dithiophosphorsäuren, z.B.:

Antimon-diamyldithiocarbamat, Zink-diamyldithiophosphat.

(f) Kombinationen von zwei oder mehr Antioxidantien der obigen, z.B.:

ein alkyliertes Amin und ein sterisch gehindertes Phenol.

Beispiele für Metallpassivatoren sind:

(a) für Kupfer, z.B. Benzotriazol, Tetrahydrobenzotriazol, 2-Mercaptobenzothiazol, 2,5-Dimercaptothiadiazol, Salicylidenpropylendiamin, Salze von Salicylaminoguanidin.

(b) für Blei, z.B. Sebacinsäurederivate, Chinizarin, Propylgallat.

(c) Kombination von zwei oder mehr der obigen Additive.

Beispiele für Rost-Inhibitoren sind:

(a) Organische Säuren, ihre Ester, Metallsalze und Anhydride, z.B.: N-Oleoyl-sarcosin, Sorbitanmono-oleat, Blei-naphthenat, Dodecenyl-bernsteinsäure-anhydrid.

(b) Stickstoffhaltige Verbindungen, z.B.:

I. Primäre, sekundäre oder tertiäre aliphatische oder cycloaliphatische Amine und Amin-Salze von organischen und anorganischen Säuren, z.B. öllösliche Alkylammoniumcarboxylate.

II. Heterocyclische Verbindungen z.B.:

Substituierte Imidazoline und Oxazoline.

(c) Phosphorhaltige Verbindungen, z.B.:

Aminsalze von Phosphorsäurepartialestern,

(d) Schwefelhaltige Verbindungen, z.B.:

Barium-dinonylnaphthalin-sulfonate, Calciumpetroleumsulfonate,

(e) Kombinationen von zwei oder mehr der obigen Additive.

Beispiele für Viskositätsindex-Verbesserer sind z.B.:

Polymethacrylate, Vinylpyrrolidon/Methacrylat-Copolymere, Polybutene, Olefin-Copolymere, Styrol/Acrylat-Copolymere.

Beispiele für Stockpunkterniedriger sind z.B.:

Polymethacrylate, Alkylierte Naphthalinderivate.

Beispiele für Dispersants/Detergents sind z.B.:

Polybutenylbernsteinsäure-imide, Polybutenylphosphonsäurederivate, überbasische Magnesium-, Calcium- und Bariumsulfonate und -phenolate.

Beispiele für andere Verschleissschutz-Additive sind z.B.

Schwefel und/oder Phosphor und/oder Halogen enthaltende Verbindungen, wie geschwefelte vegetabilische Öle, Zinkdialkyldithiophosphate, Tritolyl-phosphat, chlorierte Paraffine, Alkyl- und Aryldisulfide.

Die folgenden Beispiele erläutern die Erfindung.

Beispiel 1:
Isomerengemisch der Formel

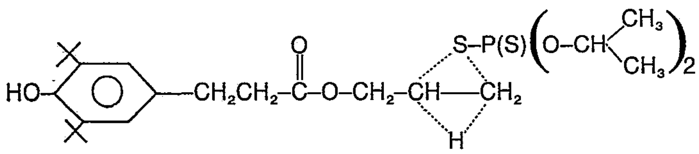

318 g (1 Mol) 3,5-Di-t-butyl-4-hydroxyphenyl-propionsäureallylester werden unter Stickstoff mit 214 g (1 Mol) Di-thiophosphorsäure-O,O-di-isopropylester versetzt und unter Zugabe von insgesamt 1 g α,α'-Azodiisobuttersäuredinitril 15 Stunden unter Rühren auf 110–120 °C erhitzt, wobei der Katalysator in 5–10 Portion, über die ganze Reaktionszeit verteilt, zugegeben wird. Es entsteht ein leicht bräunliches, dickflüssiges Reaktionsprodukt, welches nur noch wenig Allylester enthält. Die Titelverbindung ist für die erfindungsgemässe Verwendung als Schmiermittel-Additiv ohne weitere Reinigung einsetzbar, nachdem am Vakuum (2 kPa/80 °C) alle flüchtigen Nebenprodukte abgezogen wurden.

Beispiel 2:
Isomerengemisch der Formel

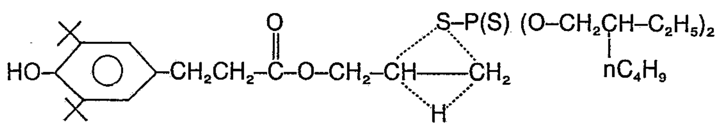

Ersetzt man den Dithiophosphorsäure-0,0-di-isopropylester von Beispiel 1 durch 353 g (1 Mol) des entsprechenden O,O-Di-2-äthyl-n-hexylesters, so erhält man bei analoger Arbeitsweise auch ohne Zugabe eines Radikal-Initiators die Titelverbindung als bräunliches viskoses Öl.

Beispiel 3

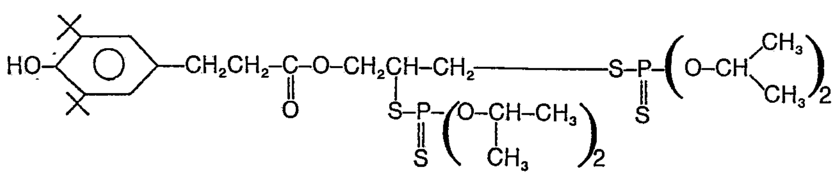

158 g (0,5 Mol) 3,5-Di-t-butyl-4-hydroxyphenyl-propionsäurepropargylester werden unter Stickstoff mit 214 g (1 Mol) Di-thio-phosphorsäure-0,0-di-isopropylester versetzt und analog Beispiel 1 mit insgesamt 1 g α,α'-Azoisobuttersäuredinitril 16 Stunden bei 110 °C unter Rühren erhitzt. Es entsteht die Titelverbindung in Form eines schwach gefärbten Öles, welches nach der Elementaranalyse zwei Dithiophosphat-Reste pro Mol enthält. Nach Entfernung flüchtiger Anteile im Vacuum ist die Substanz ohne weitere Reinigung als Schmiermitteladditiv einsetzbar.

Beispiel 4:
Isomerengemisch

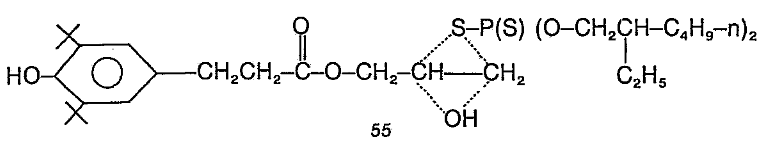

167 g (0,5 Mol) 3,5-Di-t-butyl-4-hydroxyphenyl-propionsäure-2-epoxi-propylester werden bei 20 °C unter Stickstoff und Rühren innert 30 Minuten mit 177 g (0,5 Mol) Di-thiophosphorsäure-0,0-di-2-äthyl-n-hexylester versetzt. Dabei steigt die Temperatur auf ca. 50 °C an. Die Reaktionsmischung wird noch 2 Stunden ohne weitere Wärmezuführung gerührt. Die erhaltene Titelverbindung ist ein fast farbloses, viskoses Öl.

Beispiel 7:

Mit dem Shell-Vierkugel-Apparat wurden folgende Werte bestimmt: (Tentative method IP 239/69, Extreme pressure and wear lubricant test for oils and greases, four ball-machine).

1) I.S.L.: Initial Seizure Load: Das ist die Last, bei der der Ölfilm innerhalb einer Belastungsdauer von 10 Sekunden zusammenbricht.

2) W.L. = Weld Load (Schweisslast). Das ist die Last, bei der die 4 Kugeln innerhalb von 10 Sekunden zusammenschweissen.

3) W.S.D. = Wear Scar Diameter in mm: Das ist der mittlere Verschleissdurchmesser bei einer Belastung von 70 kg bzw. 40 kg während 1 Stunde.

Als Basisöl wurde Vitrea 41 (Handelsbezeichnung der Firma Shell) verwendet.

Konzentration des Stabilisators: 1 Gew.-%.

Tabelle 1

| Stabilisator | ISL (kg) | WI (kg) | WSD (mm) |
|---|---|---|---|
| ohne | ca. 60 | ca. 160 | ca. 2,4 |
| Beispiel 1 | 140 | 250 | 0,4 |
| Beispiel 2 | 100 | 200 | 0,3 |
| Beispiel 3 | | | 0,4 |
| Beispiel 5 | | | 0,6 |

Beispiel 8:
Öl-Oxidations-Test, Standard Version nach ASTM D 2272 (Rotary Bomb Oxidation Test)

Eine Ölprobe von 50 ml Mineralöl Marke «Vitrea 41» der Fa. SHELL wird unter Zusatz von 0,25 g Stabilisator in einem Glasgefäss zusammen mit 5 ml dest. Wasser und einer blankpolierten, mit Petroläther gewaschenen katalytisch wirkenden Cu-Spirale in einer Sauerstoff-Atmosphäre oxydiert.

Das Glasgefäss befindet sich in einer Bombe aus rostfreiem Stahl mit Manometer. Die Bombe dreht sich axial mit 100 U/Min. in einem Winkel von 30° zur Horizontalen, in einem Ölbad bei 150 °C. Der Sauerstoffdruck beträgt anfangs, vor dem Aufheizen ca. 6 bar, steigt bei 150 °C auf knapp 14 bar und bleibt bis zur einsetzenden Oxidation konstant. Die Prüfung ist beendet bei einem Druckabfall um 1,7 bar. Aufgezeichnet wird die Zeit in Minuten.

Tabelle 2

| Stabilisator | Minuten bis Druckabfall um 1,7 bar |
|---|---|
| ohne | 16 |
| Beispiel 1 | 162 |
| Beispiel 2 | 70 |
| Beispiel 3 | 158 |
| Beispiel 5 | 102 |

Beispiel 9:
Öl-Oxidationstest nach IP 280, «CIGRE»

Modifizierte Version mit löslichem Cu- und Fe-Katalysator.

Bedingungen: 4 Stunden Sauerstoff einleiten bei 150 °C (4 Liter $O_2$/h).

Bestimmung der Säurezahl nach Testende; Tabellenwert: mg KOH-Verbrauch pro g Testöl.

Stabilisatorkonzentration: 0,5 Gew.-%

Testöl: Mineralöl der Marke «Vitrea 41» der Fa. SHELL.

Tabelle 3

| Stabilisator | mg KOH/g |
|---|---|
| ohne | 3,6 |
| Beispiel 1 | 1,25 |
| Beispiel 2 | 0,7 |
| Beispiel 3 | 0,9 |

**Patentansprüche**

1. Verbindungen der allgemeinen Formel I

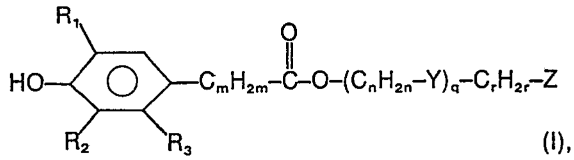

(I),

worin $R_1$ und $R_2$ unabhängig voneinander $C_1$–$C_{12}$ Alkyl, gegebenenfalls mit 1 bis 3 Alkylgruppen mit insgesamt 1 bis 12 C-Atomen substituiertes Phenyl, $C_7$–$C_9$ Aralkyl oder $C_5$–$C_7$ Cycloalkyl bedeuten, und $R_2$ zusätzlich Wasserstoff oder Chlor ist, und $R_3$ Wasserstoff oder Methyl bedeutet, und m 0, 1, 2, 3 oder 4 ist, und n und r unabhängig voneinander eine ganze Zahl von 0 bis 20 und q 1, 2 oder 3 bedeutet, mit der Bedingung, dass $(n \cdot q) + r$ eine ganze Zahl von 0 bis 24 ist, und Y eine Gruppe der Formel II oder III

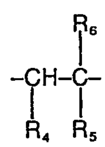

(II),

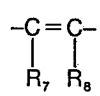

(III)

bedeutet, worin $R_4$ und $R_5$ Wasserstoff oder Hydroxy sind, und mindestens einer der Reste $R_4$ und $R_5$ eine Gruppe der Formel IV

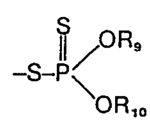

(IV)

darstellt, und $R_6$ Wasserstoff oder Methyl ist, und einer der Reste $R_7$ und $R_8$ Wasserstoff und der andere eine Gruppe der Formel IV bedeutet, wobei $R_9$ und $R_{10}$ unabhängig voneinander $C_1$–$C_{30}$ Alkyl, $C_2$–$C_{10}$ Alkoxyalkyl oder gegebenenfalls mit 1 bis 3 Alkylgruppen mit insgesamt 1 bis 12 C-Atomen substituiertes Phenyl, $C_7$–$C_9$ Aralkyl oder $C_5$–$C_7$-Cycloalkyl bedeuten, oder $R_9$ und $R_{10}$ zusammen eine Gruppe der Formel V

$$-(R_{11})C(R_{12})-[(R_{13})C(R_{14})]_t-(R_{15})C(R_{16})- \quad (V)$$

bedeuten, worin t 0 oder 1 ist und $R_{11}$, $R_{12}$, $R_{13}$, $R_{14}$, $R_{15}$ und $R_{16}$ unabhängig voneinander Wasserstoff oder Methyl sind, und Z Wasserstoff oder eine Gruppe der Formel VI

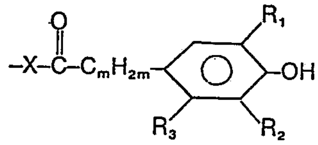

(VI),

worin die Symbole $R_1$, $R_2$, $R_3$ und m die oben angegebene Bedeutung haben, ist.

2. Verbindungen gemäss Anspruch 1 der Formel I, worin $R_1$ und $R_2$ unabhängig voneinander $C_1$–$C_8$ Alkyl sind und $R_2$ zusätzlich Wasserstoff bedeutet und $R_3$ Wasserstoff oder Methyl ist, und m 1 oder 2 ist, und n und r unabhängig voneinander eine ganze Zahl von 0 bis 8 und q 1 ist, mit der Bedingung, dass $(n \cdot q)+r$ eine ganze Zahl von 0 bis 16 bedeutet, und Y eine Gruppe der Formel II oder III ist, worin $R_4$ und $R_5$ Wasserstoff oder Hydroxy sind, und mindestens einer der Reste $R_4$ und $R_5$ eine Gruppe der Formel IV darstellt und $R_6$ Wasserstoff ist, einer der Reste $R_7$ und $R_8$ Wasserstoff und der andere eine Gruppe der Formel IV bedeutet, wobei $R_9$ und $R_{10}$ unabhängig voneinander der $C_1$–$C_{22}$ Alkyl sind, oder $R_9$ und $R_{10}$ zusammen eine Gruppe der Formel V bedeuten, worin die Symbole t und $R_{11}$ bis $R_{16}$ die im Anspruch 1 angegebene Bedeutung haben, und Z Wasserstoff ist.

3. Verbindungen gemäss Anspruch 2 der Formel I, worin $R_1$ t.-Butyl, t.-Amyl oder 1,1,3,3-Tetramethylbutyl ist und $R_2$ $C_1$–$C_8$ Alkyl bedeutet, $R_3$ Wasserstoff ist, und n eine ganze Zahl von 0 bis 6 bedeutet, und r 0 oder 1, und q 1 ist, und Y eine Gruppe der Formel II oder III ist, wobei einer der Substituenten $R_4$ und $R_5$ in Formel II bzw. $R_7$ und $R_8$ in Formel III Wasserstoff und der andere eine Gruppe der Formel IV ist, worin $R_9$ und $R_{10}$ die in Anspruch 2 angegebene Bedeutung haben.

4. Verbindungen gemäss einem der Ansprüche 1 oder 3 der Formel I, worin n 1, r 0 und q 1 sind.

5. Verbindungen gemäss Anspruch 1 der Formel I, worin Y eine Gruppe der Formel II ist.

6. Verbindungen gemäss Anspruch 1 der Formel I, worin m 1 oder 2 ist.

7. Verbindungen gemäss Anspruch 1 der Formel I, worin Z Wasserstoff ist.

8. Verbindungen gemäss Anspruch 1 der Formel I, worin q 1 ist.

9. Verbindungen gemäss Anspruch 1 der Formel I, worin r 0 ist.

10. Die Verbindung gemäss Anspruch 1 der Formel

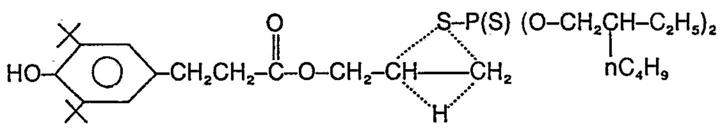

11. Organisches polymeres Material enthaltend eine Verbindung der Formel I gemäss Anspruch 1.

12. Schmiermittel enthaltend eine Verbindung der Formel I gemäss Anspruch 1.

13. 3-(3,5-Di-tert.-butyl-4-hydroxyphenyl)-propionsäureallylester.

14. 3-(3,5-Di-tert.-butyl-4-hydroxyphenyl)-propionsäurepropargylester.

## Claims

1. A compound of the general formula I

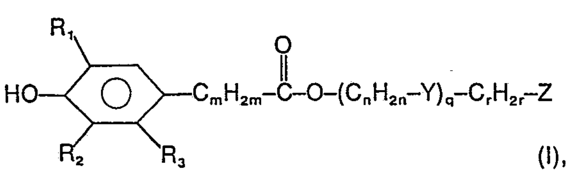

(I),

wherein $R_1$ and $R_2$ independently of one another are each $C_1$–$C_{12}$-alkyl, or phenyl which is unsubstituted or substituted by 1 to 3 alkyl groups having a total of 1 to 12 C atoms, or are each $C_7$–$C_9$-aralkyl on $C_5$–$C_7$-cycloalkyl, and $R_2$ is additionally hydrogen or chlorine, $R_3$ is hydrogen or methyl, m is nought, 1, 2, 3 or 4, n and r independently of one another are each an integer from nought to 20, q is 1, 2 or 3, with the proviso that $n \cdot q)+r$ is an integer from nought to 24, and Y is a group of the formula II or III

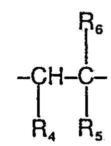

(II),

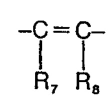

(III)

in which $R_4$ and $R_5$ are each hydrogen or hydroxyl, and at least one of the radicals $R_4$ and $R_5$ is a group of the formula IV

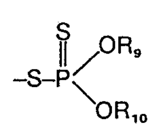

(IV)

$R_6$ is hydrogen or methyl, one of the radicals $R_7$ and $R_8$ is hydrogen and the other is a group of the formula IV, wherein $R_9$ and $R_{10}$ independently of one another are each $C_1$–$C_{30}$-alkyl, $C_2$–$C_{10}$-alkoxyalkyl, or phenyl which is unsubstituted or substituted by 1 to 3 alkyl groups having a total of 1 to 12 C atoms, or are each $C_7$–$C_9$-aralkyl or $C_5$–$C_7$-cycloalkyl, or $R_9$ and $R_{10}$ together are a group of the formula V

$-(R_{11})C(R_{12})-[(R_{13})C(R_{14})]_t-(R_{15})C(R_{16})-$ (V)

wherein t is nought or 1, and $R_{11}$, $R_{12}$, $R_{13}$, $R_{14}$, $R_{15}$ and $R_{16}$ independently of one another are each hydrogen or methyl, and Z is hydrogen or a group of the formula VI

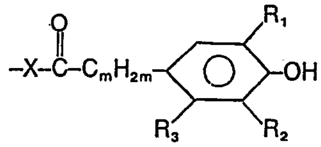

(VI),

in which the symbols $R_1$, $R_2$, $R_3$ and m have the meanings defined above.

2. A compound according to Claim 1 of the formula I, wherein $R_1$ and $R_2$ independently of one another are each $C_1$–$C_8$-alkyl, with $R_2$ in addition being hydrogen, $R_3$ is hydrogen or methyl, m is 1 or 2, n and r independently of one another are each an integer from nought to 8 inclusive, q is 1, with the proviso that $(n \cdot q)+r$ is an integer from nought to 16 inclusive, Y is a group of the formula II or III wherein $R_4$ and $R_5$ are each hydrogen or hydroxyl, and at least one of the radicals $R_4$ and $R_5$ is a group of the formula IV, $R_6$ is hydrogen, one of the radicals $R_7$ and $R_8$ is hydrogen and the other is a group of the formula IV, $R_9$ and $R_{10}$ independently of one another being $C_1$–$C_{22}$-alkyl, or $R_9$ and $R_{10}$ together are a group of the formula V, wherein the symbols t and $R_{11}$ to $R_{16}$ have the meanings given in Claim 1, and Z is hydrogen.

3. A compound according to Claim 2 of the formula I, wherein $R_1$ is t-butyl, t-amyl or 1,1,3,3-tetramethylbutyl, $R_2$ is $C_1$–$C_8$-alkyl, $R_3$ is hydrogen, n is an integer from nought to 6, r is nought or 1, q is 1, Y is a group of the formula II or III, in which one of the substituents $R_4$ and $R_5$ in the formula II or $R_7$ and $R_8$ in formula III is hydrogen and the other is a group of the formula IV, wherein $R_9$ and $R_{10}$ have the meanings given in Claim 2.

4. A compound according to one of the Claims 1 or 3 of the formula I wherein n is 1, r is nought, and q is 1.

5. A compound according to Claim 1 of the formula I wherein Y is a group of the formula II.

6. A compound according to Claim 1 of the formula I wherein m is 1 or 2.

7. A compound according to Claim 1 of the formula I wherein Z is hydrogen.

8. A compound according to Claim 1 of the formula I wherein q is 1.

9. A coumpound according to Claim 1 of the formula I wherein r is nought.

10. A compound according to Claim 1 of the formula

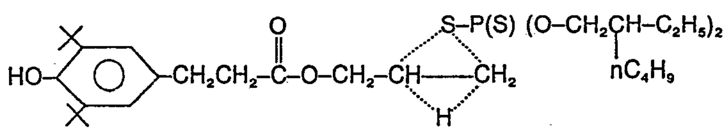

11. Organic polymeric material containing a compound of the formula I according to Claim 1.

12. A lubricant containing a compound of the formula I according to Claim 1.

13. Allyl 3-(3,5-di-tert-butyl-4-hydroxyphenyl) propionate.

14. Propargyl 3-(3,5-di-tert-butyl-4-hydroxyphenyl)propionate.

**Revendications**

1. Composés répondant à la formule générale I

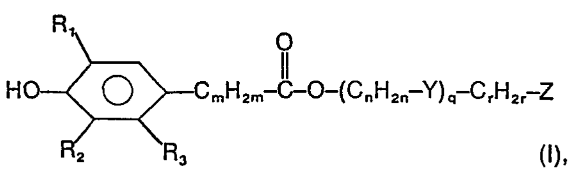

(I),

dans laquelle

$R_1$ et $R_2$ représentent chacun, indépendamment l'un de l'autre, un alkyle en $C_1$–$C_{12}$, un radical phényle, aralkyle en $C_7$–$C_9$ ou cycloalkyle en $C_5$–$C_7$ éventuellement porteur d'1 à 3 radicaux alkyles contenant au total de 1 à 12 atomes de carbone, $R_2$ pouvant en outre représenter l'hydrogène ou le chlore,

$R_3$ représente l'hydrogène ou un méthyle,

m représente un nombre égal à 0, à 1, à 2, à 3 ou à 4,

n et r représentent chacun, indépendamment l'un de l'autre, un nombre entier de 0 à 20,

q représente un nombre égal à 1, à 2 ou à 3 avec la condition que la somme $(n \cdot q)+r$ soit un nombre entier de 0 à 24,

Y représente un radical répondant à l'une des formules II et III:

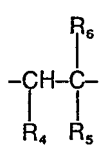

(II),

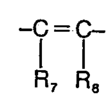

(III)

dans lesquelles $R_4$ et $R_5$ représentent chacun l'hydrogène ou un groupe hydroxy, au moins l'un de ces symboles $R_4$ et $R_5$ désignant un radical de formule IV:

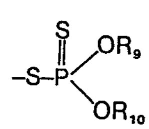

(IV)

$R_6$ représente l'hydrogène ou un méthyle, l'un des symboles $R_7$ et $R_8$ représente l'hydrogène et l'autre un radical de formule IV, $R_9$ et $R_{10}$ représentent chacun, indépendamment l'un de l'autre, un al-

kyle en $C_1$–$C_{30}$, un alcoxyalkyle en $C_2$–$C_{10}$, un radical phényle, aralkyle en $C_7$–$C_9$ ou cycloalkyle en $C_5$–$C_7$ éventuellement porteur d'un à trois radicaux alkyles contenant au total de 1 à 12 atomes de carbone, ou encore $R_9$ et $R_{10}$ forment ensemble un radical répondant à la formule V:

$$-(R_{11})C(R_{12})-[(R_{13})C(R_{14})]_t-(R_{15})C(R_{16})- \quad (V)$$

dans laquelle t est un nombre égal à 0 ou à 1 et $R_{11}$, $R_{12}$, $R_{13}$, $R_{14}$, $R_{15}$ et $R_{16}$ représentent chacun, indépendamment les uns des autres, l'hydrogène ou un méthyle, et

Z représente l'hydrogène ou un radical répondant à la formule VI

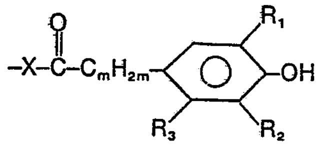

(VI),

dans laquelle $R_1$, $R_2$, $R_3$ et m ont les significations indiquées plus haut.

2. Composés de formule I selon la revendication 1, dans lesquels $R_1$ et $R_2$ représentent chacun, indépendamment l'un de l'autre, un alkyle en $C_1$–$C_8$, $R_2$ pouvant en outre représenter l'hydrogène, $R_3$ représente l'hydrogène ou un méthyle, m est égal à 1 ou à 2, n et r représentent chacun, indépendamment l'un de l'autre, un nombre entier de 0 à 8, q est égal à 1, avec la condition que la somme $(n \cdot q) + r$ soit égale à un nombre entier de 0 à 16, et Y représente un radical de formule II ou III dans lequel $R_4$ et $R_5$ représentent l'hydrogène ou un hydroxy et au moins l'un d'eux représente un radical de formule IV, $R_6$ représente l'hydrogène, l'un des symboles $R_7$ et $R_8$ représente l'hydrogène et l'autre un radical de formule IV, les symboles $R_9$ et $R_{10}$ présents dans cette formule IV représentant chacun, indépendamment l'un de l'autre, un alkyle en $C_1$–$C_{22}$ ou encore formant ensemble un radical de formule V dans lequel les symboles t et $R_{11}$ à $R_{16}$ ont les significations précédemment données, et Z représente l'hydrogène.

3. Composés de formule I selon la revendication 2, dans lesquels $R_1$ représente un radical tert-butyle, tert-pentyle ou tétraméthyl-1,1,3,3 butyle, $R_2$ représente un alkyle en $C_1$–$C_8$, $R_3$ représente l'hydrogène, n désigne un nombre entier de 0 à 6, r est égal à 0 ou à 1, q est égal à 1, et Y représente un radical de formule II ou de formule III, l'un des symboles $R_4$ et $R_5$ de la formule II, ou $R_7$ et $R_8$ de la formule III, représentant l'hydrogène et l'autre radical de formule IV dans lequel $R_9$ et $R_{10}$ ont les significations données à la revendication 2.

4. Composés de formule I selon l'une des revendications 1 et 3, dans lesquels n est égal à 1, r à 0 et q à 1.

5. Composés de formule I selon la revendication 1, dans lesquels Y représente un radical de formule II.

6. Composés de formule I selon la revendication 1, dans lesquels m est égal à 1 ou à 2.

7. Composés de formule I selon la revendication 1, dans lesquels Z représente l'hydrogène.

8. Composés de formule I selon la revendication 1, dans lesquels q est égal à 1.

9. Composés de formule I selon la revendication 1, dans lesquels r est égal à 0.

10. Composé selon la revendication 1, en l'espèce celui qui répond à la formule suivante:

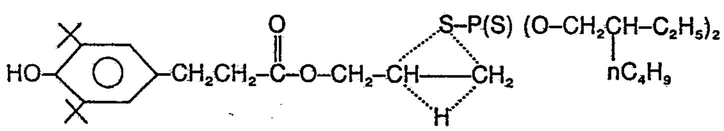

11. Matière organique polymère qui contient un composé de formule I selon la revendication 1.

12. Lubrifiant qui contient un composé de formule I selon la revendication 1.

13. (Ditert-butyl-3,5 hydroxy-4 phényl)-3 propionate d'allyle.

14. (Ditert-butyl-3,5 hydroxy-4 phényl)-3 propionate de propargyle.